# EUROPÄISCHE PATENTSCHRIFT

(11) **EP 2 150 230 B1**
(45) Veröffentlichungstag und Bekanntmachung des Hinweises auf die Patenterteilung: **13.04.2016**
(21) Anmeldenummer: 07856588.4
(22) Anmeldetag: 12.12.2007
(51) Int. Cl.: A61Q 19/00, A61K 8/37, A61Q 19/08, A61Q 19/10, A61Q 5/02

(54) **REINIGENDE ÖI-IN-WASSER-EMULSIONEN**
CLEANSING OIL-IN-WATER EMULSIONS
ÉMULSIONS HUILE DANS L'EAU NETTOYANTES

(30) Priorität: 28.02.2007 DE 102007010108; 10.12.2007 DE 102007059704
(43) Veröffentlichungstag der Anmeldung: 10.02.2010
(73) Patentinhaber: Henkel AG & Co. KGaA, 40589 Düsseldorf (DE)
(72) Erfinder: SCHELGES, Heike, 47877 Willich (DE); KREISIG, Annette, NL-6291 CJ Vaals (DE)
(86) Internationale Anmeldenummer: PCT/EP2007/010840
(87) Internationale Veröffentlichungsnummer: WO 2008/104214

(56) Entgegenhaltungen:
- EP-A- 0 163 806
- EP-A- 1 889 641
- WO-A-2005/097044
- WO-A-2005/097055
- DE-A1-102004 031 939
- US-A1- 2002 035 046
- US-A1- 2004 167 046

## Beschreibung

Die Erfindung betrifft Öl-in-Wasser-Formulierungen und deren Verwendung als Hautpflegemittel, insbesondere als Hautreinigungsmittel und insbesondere als reinigendes Antifalten-Mittel und/oder reinigendes Anti-Akne-Mittel.

### Stand der Technik

Milde Reinigungszusammensetzungen sind dem Fachmann seit langem bekannt.
US 2004/0167046 A1 offenbart eine Öl-in-Wasser-Emulsion mit Pentaerythrityltetraoctanoat, einem bei Raumtemperatur flüssigen Öl mit einem Schmelzpunkt unter 30 °C. US 2002/035046 A1 offenbart eine reinigende Öl-in-Wasser-Emulsion mit Pentaerythrityltetraoctanoat, einem bei Raumtemperatur flüssigen Öl mit einem Schmelzpunkt unter 30 °C, und PEG-150 Pentaerythrityltetrastearat. EP 163806 A1 offenbart kosmetische Zusammensetzungen, die als Moisturizer, Emollient und Emulsionsstabilisator bei Raumtemperatur flüssige Oligomere von teilweise verestertem Pentaerythritol enthalten. EP 163806 A1 offenbart spezifisch eine Reinigungscreme mit Tripentaerythrityltetralaurat/myristat.

Aufgabe der vorliegenden Erfindung war es, Reinigungszusammensetzungen zur Verfügung zu stellen, die mild sind.
Eine weitere Aufgabe war es, Reinigungszusammensetzungen zu formulieren, die ein angenehmes, sensorisch leichtes Profil und gute Pflegeeffekte aufweisen und bei Applikation auf der Haut nicht brechen.
Ein weiterer Aspekt der Aufgabe war es, möglichst irritationsfreie Reinigungszusammensetzungen bereitzustellen.

Überraschend wurde nun gefunden, dass sich diese Eigenschaften erreichen lassen, wenn man Öl-in-Wasser-Emulsionen formuliert, die eine erfindungsgemäße Kombination von ausgewählten Wachsen und Ölen enthalten.

### Beschreibung

Gegenstand der vorliegenden Anmeldung ist die Verwendung einer Haut reinigenden Öl-in-Wasser-Emulsion, enthaltend (a) 1 - 10 Gew.-% wenigstens einer Wachskomponente mit einem Schmelzpunkt von wenigstens 30°C, ausgewählt aus der Gruppe der Pentaerythritester und/oder der Dipentaerythritester sowie Mischungen hiervon, wobei die Wachskomponente ausgewählt ist aus der Gruppe der Ester, die durch Umsetzung des Pentaerythrits oder des Dipentaerythrits mit einem Fettsäuregemisch, enthaltend 40 - 50 Gew.-% C16-Fettsäure und 45 - 55 Gew.-% C18-Fettsäure, erhältlich ist, (b) 1 - 30 Gew.-% wenigstens einer bei 25°C flüssigen Ölkomponente, (c) 40 - 95 Gew.-% Wasser und (d) nichtionische Tenside/Emulgatoren In einer Gesamtmenge von 0 - 0,7 Gew.-%, bezogen auf das Gesamtgewicht der Emulsion, zur Reinigung der Haut, insbesondere zur Reinigung der Gesichtshaut.

Zusammensetzungen dieser Art sind langzeitstabil und hinterlassen bei der Anwendung ein glattes und welches Gefühl mit sehr guten Pflegeeigenschaften. Sie sind leicht verteilbar, ziehen gut auf die Haut auf, hinterlassen ein geringes öliges oder fettendes, sondern vielmehr samtiges Hautgefühl.
In einer bevorzugten Ausführungsform enthalten die erfindungsgemäß verwendeten Öl-in-Wasser-Emulsionen keine zusätzlichen anionischen und kationischen Tenside/Emulgatoren. Dadurch haben die Zusammensetzungen ein geringes Irritationspotenzial.

Überraschend wurde gefunden, dass die erfindungsgemäß verwendeten Öl-in-Wasser-Emulsionen gemäß Anspruch 1 auch ohne die Anwesenheit von Tensiden/Emulgatoren, insbesondere auch ohne nichtionische Tenside/Emulgatoren, eine gute Reinigungswirkung haben. Die Anwesenheit geringer Mengen an Tensiden/Emulgatoren, Insbesondere an nichtionischen Tensiden/Emulgatoren, kann gegebenenfalls erforderlich sein, um Parfümöle oder andere ölähnliche Substanzen zu solubilisieren. Auch können geringe Mengen an Tensiden/Emulgatoren eingetragen werden durch gelbildende Polymerzubereitungen (siehe Abschnitt "Gelbildner"). Die erfindungsgemäß verwendeten Öl-in-Wasser-Emulsionen enthalten nichtionische Tenside/Emulgatoren in einer Gesamtmenge von 0 - 0,7 Gew.-%, bevorzugt 0 - 0,5 Gew.-%, besonders bevorzugt 0 - 0,3 Gew.%, außerordentlich bevorzugt 0 - 0,1 Gew.-%, jeweils bezogen auf das Gesamtgewicht der Emulsion. Dadurch haben die Zusammensetzungen ein weiter minimiertes Irritationspotenzial.

Die erfindungsgemäß verwendeten O/W-Emulsionen weisen vorzugsweise eine Viskosität von 20000 bis 500000 mPa s, bevorzugt 50000 bis 80000 mPa·s, auf, gemessen bei 20°C mit einem Bronkfield-Viskosimeter RVF, Spindel TE, mit Helipath bei 4 Umdrehungen pro Minute.

Pentaerythritester, Dipentaerythritester und/oder Tripentaerythritester als Wachskomponente Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30 °C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß obligatorisch ist eine Wachskomponente oder ein Gemisch von Wachskomponenten, die bei 30 °C oder darüber schmilzt/schmelzen und ausgewählt ist/sind aus der Gruppe der Ester des Pentaerythrits und des Dipentaerythrits. Die Ester des Pentaerythrits sind bevorzugt. Die vorgenannten Ester des Pentaerythrits und/oder Dipentaerythrits sind in den erfindungsgemäß verwendeten Öl-in-Wasser-Emulsionen in einer Gesamtmenge von 1 - 10 Gew.-% enthalten. In einer bevorzugten Ausführungsform der Erfindung liegt der Gehalt dieser Pentaerythrit-basierten Wachskomponente bei 1 - 5 Gew.-%, besonders bevorzugt bei 1 - 4 Gew.-% und insbesondere 1 - 2 Gew.-%, jeweils bezogen auf die Gesamtzusammensetzung. In den genannten Mengenbereichen ist das sensorische Gesamtprofil der erfindungsgemäß verwendeten Öl-in-Wasser-Emulsionen optimal.
Eine weitere bevorzugte Ausführungsform der erfindungsgemäß verwendeten Öl-in-Wasser-Emulsionen ist dadurch gekennzeichnet, dass die Wachskomponente oder das Gemisch aus Wachskomponenten (a) einen Schmelzpunkt zwischen 40 °C und 80 °C aufweist, vorzugsweise zwischen 40 und 60 °C, da sich in diesem Bereich die besten sensorischen Effekte ergeben.

Erfindungsgemäß verwendete O/W-Emulsionen sind dadurch gekennzeichnet, dass die Wachskomponente (a) ausgewählt ist aus der Gruppe der Ester, die durch Umsetzung des Pentaerythrits oder Dipentaerythrits mit einem Fettsäuregemisch, enthaltend 40 - 50 Gew.-% C16-Fettsäure und 45 - 55 Gew.-% C18-Fettsäure, erhältlich sind. Besonders bevorzugt sind Ester linearer, unverzweigter C16/C18-Fettsäuren.
Eine weitere bevorzugte Ausführungsform der erfindungsgemäß verwendeten Öl-in-Wasser-Emulsionen enthält wenigstens eine Wachskomponente (a), ausgewählt aus der Gruppe der Ester des Pentaerythrits mit einem Anteil an 5 - 35 Gew.-% Monoester, 20 - 50 Gew.-% Diester und 25 - 50 Gew.-% Triester, und ggf. Tetraester. Ganz besonders bevorzugt sind Ester, die durch Umsetzung des Pentaerythrits mit einem Fettsäuregemisch, enthaltend 40 - 50 Gew.-% C16-Fettsäure und 45 - 55 Gew.-% C18-Fettsäure, erhältlich sind und die folgende Esterverteilung aufweisen: 12 - 19 Gew.-% Monoester, 25 - 35 Gew.-% Diester, 30 - 40 Gew.-% Triester und 6 - 11 Gew.-% Tetraester.
Erfindungsgemäß besonders bevorzugt ist das Handelsprodukt Cutina PES (ex Cognis), INCI-Bezeichnung Pentaerythrityl Distearate

### Ölkörper

Die erfindungsgemäß verwendeten Öl-in-Wasser-Emulsionen enthalten weiterhin obligatorisch 1 - 30 Gew.-%, bezogen auf die Gesamtzusammensetzung, mindestens einer bei 25°C flüssigen Ölkomponente. Vorzugsweise ist/sind die Ölkomponente(n) in einer Gesamtmenge von 3 - 20 Gew.-%, insbesondere 5 - 15 Gew.-% und besonders bevorzugt 7 - 12 Gew.-%, bezogen auf das Gesamtgewicht der erfindungsgemäß verwendeten Öl-in-Wasser-Emulsion, enthalten.
Als Ölkörper sind beispielsweise die nachstehend genannten Verbindungsklassen geeignet, soweit diese bei 25°C flüssig sind. Hierzu zählen u.a. Guerbetalkohole auf Basis von Fettalkoholen mit 6 - 18, vorzugsweise 8 - 10 Kohlenstoffatomen, Ester von linearen oder verzweigten, gesättigten oder ungesättigten C6-C22-Fettsäuren mit linearen oder verzweigten, gesättigten oder ungesättigten C6-C22-Fettalkoholen, insbesondere 2-Ethylhexanol. Beispielhaft seien genannt Hexyllaurat, Myristylisostearat, Myristyloleat, Cetylisostearat, Cetyloleat, Stearylisostearat, Stearyloleat, Isostearylisononanoat, Isostearylmyristat, Isostearylpalmitat, Isostearylstearat, Isostearylisostearat, Isostearyloleat, Oleylmyristat, Oleylisostearat, Oleyloleat, Oleylerucat, Erucylisostearat, Erucyloleat, Cococaprylat/caprat. Weitere geeignete Ester sind z.B. Ester von C18-C38-Alkylhydroxycarbonsäuren mit linearen oder verzweigten, gesättigten oder ungesättigten C6-C22-Fettalkoholen, Ester von linearen und/oder verzweigten, gesättigten oder ungesättigten Fettsäuren mit mehrwertigen Alkoholen (wie z. B. Propylenglycol, Dimerdiol oder Trimertriol) und/oder Guerbetalkoholen, weiterhin Triglyceride und Triglyceridmischungen, Mono-/ Di-/Triglyceridmischungen, Ester von C6-C22-Fettalkoholen und/oder Guerbetalkoholen mit aromatischen Carbonsäuren, insbesondere Benzoesäure, Ester von C2-C12-Dicarbonsäuren mit linearen oder verzweigten, gesättigten oder ungesättigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare Dialkylcarbonate, Guerbetcarbonate auf Basis von Fettalkoholen mit 6 bis 18, vorzugsweise 8 bis 10 C-Atomen, Ester der Benzoesäure mit linearen und/oder verzweigten C6-C22-Alkoholen (z. B. Cetiol AB), lineare oder verzweigte symmetrische oder unsymmetrische Dialkylether mit 6 bis 22 Kohlenstoffatomen pro Alkylgruppe, wie z. B. Di-n-octyl-ether (Cetiol OE), Ringöffnungsprodukte von epoxidierten Fettsäureestern mit Polyolen, Kohlenwasserstoffe wie Paraffin- oder Mineralöle, Siliconöle, Oligo-oder Polyalphaolefine, Dialkylcarbonate, Ester aus C8-C24-Fettsäuren und C8-C24-Fettalkoholen, sowie Mischungen dieser Substanzen.
Eine erfindungsgemäß bevorzugte Gruppe von Ölen sind die Dialkylcarbonate. Die Dialkylcarbonate können symmetrisch oder unsymmetrisch, verzweigt oder unverzweigt, gesättigt oder ungesättigt sein und lassen sich nach Umesterungsreaktionen, die aus dem Stand der Technik hinlänglich bekannt sind, herstellen. Erfindungsgemäß besonders geeignet sind Dialkylcarbonate mit Alkylketten, die 6 bis 24 Kohlenstoffatome aufweisen, insbesondere Di-n-octylcarbonat oder Di-(2-ethylhexyl)carbonat oder ein Gemisch dieser Substanzen. Unter diesen ist das Di-n-Octylcarbonat bevorzugt.
Weitere bevorzugte Öle sind ausgewählt aus den Estern von linearen oder verzweigten, gesättigten oder ungesättigten C6-C22-Fettsäuren mit linearen oder verzweigten, gesättigten oder ungesättigten C6-C22-Fettalkoholen, insbesondere Hexyllaurat, Isostearylisononanoat, Isostearylpalmitat, Isostearylstearat, Isostearylisostearat und Cococaprylat/caprat.
Weitere bevorzugte Öle sind ausgewählt aus den Triglyceriden, das heißt, den Glyceryltriestern, und Triglyceridmischungen, die bei 25 °C flüssig sind. Hierzu zählen besonders bevorzugt die Glyceryltriester der Octansäure (Caprylic Triglyceride) und der Decansäure (Capric Triglyceride). Weitere bevorzugte Öle sind ausgewählt aus pflanzlichen Ölen, insbesondere Distelöl, Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls. Ein aufgrund seiner relativ starken Polarität besonders bevorzugtes pflanzliches Öl ist Distelöl.
Weitere bevorzugte Öle sind ausgewählt aus den Estern der Benzoesäure mit linearen und/oder verzweigten C6-C22-Alkoholen, insbesondere aus Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD.
Weitere bevorzugte Öle sind ausgewählt aus den verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16, Guerbitol^{®} T 16), Octyldodecanol (Eutanol^{®} G, Guerbitol^{®} 20), 2-Ethylhexylalkohol und die Handelsprodukte Guerbitol^{®} 18, Isofol^{®} 12, Isofol^{®} 16, Isofol^{®} 24, Isofol^{®} 36, Isocarb^{®} 12, Isocarb^{®} 16 oder Isocarb^{®} 24, wobei Hexyldecanol besonders bevorzugt ist.

Mit Ausnahme der Kohlenwasserstoffe, wie Paraffin- oder Mineralöle, Oligo- oder Polyalphaolefine, und der Siliconöle werden die übrigen vorstehend aufgeführten Substanzklassen eher zu den polaren Ölen gerechnet. Kohlenwasserstoffe, wie Paraffin- oder Mineralöle, Oligo- oder Polyalphaolefine, und die Siliconöle zählen dagegen eher zu den unpolaren Ölen. Überraschend wurde festgestellt, dass sich besonders lager- und temperaturstabile Öl-in-Wasser-Emulsionen formulieren lassen, wenn die Ölkomponente mindestens ein polares Öl und mindestens ein unpolares Öl enthält, wobei die polaren Öle gegenüber den unpolaren Ölen im Überschuss vorliegen. Bevorzugt beträgt dabei das Gewichtsverhältnis der Gesamtheit der polaren Öle zur Gesamtheit der unpolaren Öle 5:1 bis 20:1, bevorzugt 6:1 - 10:1 und besonders bevorzugt 7:1 - 8:1.

Erfindungsgemäß besonders bevorzugte polare Öle sind ausgewählt aus Di-n-Octylcarbonat, Cococaprylat/caprat, Hexyllaurat, Isostearylisononanoat, Isostearylpalmitat, Isostearylstearat, Isostearylisostearat, Caprylic Triglyceride, Capric Triglyceride, Distelöl, Benzoesäure-C12-C15-alkylester, Hexyldecanol, Octyldodecanol, sowie Mischungen dieser Öle.
Eine besonders bevorzugte Ölmischung umfasst Di-n-Octylcarbonat und Cococaprylat/caprat. Überraschend wurde festgestellt, dass diese Ölmischung die Reinigungswirkung besonders vorteilhaft unterstützt und darüber hinaus nicht-komedogen wirkt, was insbesondere vorteilhaft für das bevorzugte Anwendungsgebiet der erfindungsgemäßen Zusammensetzungen ist, nämlich die Behandlung unreiner Haut oder Aknehaut. Als nicht-komedogene Ölkörper gelten ansonsten eher die Siliconöle, die aber nicht zu den polaren Ölen gerechnet werden.

Diese polaren Öle und Ölmischungen werden erfindungsgemäß besonders bevorzugt mit Polydimethylsiloxanen (INCI: Dimethicone) als unpolarer Ölkomponente kombiniert.

Weitere erfindungsgemäß als eher unpolare Öle einsetzbare Kohlenwasserstoffe weisen eine Kettenlänge von vorzugsweise 8 bis 40 C-Atomen aus. Sie können verzweigt oder unverzweigt sein, gesättigt oder ungesättigt. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatomen aufweisen, sind besonders geeignet, und unter diesen ist ein Gemisch aus Alkanen, das wenigstens 10 Gew.-% verzweigte Alkane, bezogen auf die Gesamtmenge der Alkane, enthält, besonders bevorzugt. Bevorzugt handelt es sich um verzweigte, gesättigte Alkane. Besonders gut geeignet sind Gemische aus Alkanen, die mehr als 1 Gew.-% 5,8-Diethyldodecan und/oder mehr als 1 Gew.-% Didecen enthalten.

### Retinoide

Erfindungsgemäß bevorzugt verwendete Öl-in-Wasser-Emulsionen sind gekennzeichnet durch den Gehalt an mindestens einem Retinoid. Dieses unterstützt die Reinigungsleistung insbesondere bei unreiner oder Aknehaut. Weiterhin stellt es einen zusätzlichen Benefit für Reinigungsmittel mit Antifaltenwirkung dar.
Zu den Retinoiden werden im Sinne der vorliegenden Erfindung Retinol (Vitamin A₁), die C₂-C₂₂-Fettsäureester des Retinols (= Retinylester, insbesondere Retinylpalmitat und Retinylacetat), 3,4-Didehydroretinol (Vitamin A₂), Retinal und seine Isomere, *all-trans*-Retinsäure, 9-*cis*-Retinsäure und 13-*cis*-Retinsäure (Tretinoin), die Ester der Retinsäure sowie weitere verwandte Substanzen (synthetische Retinoide) gezählt, die für ihre vielfältige biologische Wirkung insbesondere auf Wachstum und Differenzierung bekannt sind, sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen. Synthetische Retinoide werden in drei Gruppen eingeteilt: nichtaromatisch (z. B. *Isotretinoin*)*,* monoaromatisch (z.B. Acitretin) und polyaromatisch (so genannte Arotinoide, z. B. Tazarotene, die eine spezifische Wirkung auf einzelne Retinoid-Rezeptoren haben). Erfindungsgemäß besonders bevorzugte Retinoide sind Retinol und die C₂-C₂₂-Fettsäureester des Retinols, insbesondere Retinylpalmitat und Retinylacetat.
Retinoide werden bevorzugt als Antifaltenwirkstoff und/oder als Anti-Akne-Wirkstoff und/oder als Wirkstoff gegen unreine Haut verwendet.

Erfindungsgemäß besonders bevorzugt verwendete Öl-in-Wasser-Emulsionen enthalten mindestens ein Retinoid in einer Gesamtmenge von 0,01 - 2 Gew.-%, bevorzugt 0,02 - 1 Gew.-%, besonders bevorzugt 0,05 - 0,5 Gew.-%, außerordentlich bevorzugt 0,07 - 0,1 Gew.-%, jeweils bezogen auf die gesamte Öl-in-Wasser-Emulsion.

### Gelbildner

Erfindungsgemäß bevorzugte Öl-in-Wasser-Emulsionen enthalten mindestens einen Gelbildner, bevorzugt einen Hydrogelbildner, der besonders bevorzugt aus der Gruppe der Homopolymere oder Copolymere von Acrylsäure und/oder Acrylamid und/oder Acryloyldimethyltaurat und deren Salzen oder aus einem beliebigen Gemisch dieser Substanzen ausgewählt ist. Häufig sind die marktüblichen Hydrogelbildner als wasserbasierte Polymerzubereitungen konfektioniert, die gegebenenfalls ein nichtionisches Tensid/Emulgator und gegebenenfalls ein Öl enthalten. Zu den erfindungsgemäß bevorzugten Gelbildnern gehören im Handel erhältliche Substanzen oder Polymerzubereitungen, wie beispielsweise Sepigel 305, INCI: Polyacrylamid (und) C13-14 Isoparaffin (und) Laureth-7; Sepigel 501, INCI: Acrylamid Copolymer (und) Mineralöl (und) C13-14 Isoparaffin (und) Polysorbate 85, Sepigel 502, INCI: C13-14 Isoparaffin (und) Isostearyl Isostearat (und) Sodium Polyacrylate (und) Polyacrylamid (und) Polysorbate 60; Simulgel 600, INCI: Acrylamid/Sodium Acryloyldimethyltaurate Copolymer (und) Isohexadecan (und) Polysorbat 80; Simulgel 800, INCI: Sodium Polyacryloyldimethyl Taurate (und) Isohexadecan (und) Sorbitan Oleat; Simulgel EG, INCI: Sodium Acrylate/Acryloyldimethyl Taurate Copolymer (und) Isohexadecan (und) Polysorbate 80; Simulgel EG-SL (oder auch Simulgel EPG), INCI: Sodium Acrylate/Acryloyldimethyl Taurate Copolymer (und) Polyisobutene (und) Caprylyl/Capryl Glucosid; Simulgel NS, INCI: Hydroxyethyl Acrylate (und) Sodium Acryloyldimethyl Taurate Copolymer (und) Squalane (und) Polysorbate 60; Aristoflex AVC, INCI: Ammonium Acryloyldimethyltaurate/VP Copolymer; Aristoflex AVC-1, INCI: Ammonium Acryloyldimethyltaurat / Vinyl Formamid Copolymer; Aristoflex HMB, INCI: Ammonium Acryloyldimethyltaurate / Beheneth-25 Methacrylate Copolymer, Salcare SC 91, INCI: Sodium Acrylate Copolymer (und) Mineralöl (und) PPG-1 Trideceth-6; Salcare AST, INCI: Sodium Acrylate Copolymer (und) Glycine Soja (und) PPG-1 Trideceth-6; Pemulen TR-1, INCI: Acrylates / C10-30 Alkyl Acrylate Crosspolymer; Pemulen TR-2: Acrylates / C10-30 Alkyl Acrylate Crosspolymer; Carbopol 980, INCI: Carbomer (z.B. Homopolymere von Acrylsäure, vernetzt mit einem Allylether von Pentaerythritol, einem Allylether von Sucrose oder einem Allylether von Propylen); Carbopol ETD 2020, INCI: Acrylates / C10-30 Alkyl Acrylate Crosspolymer; Carbopol Ultrez 10, INCI: Carbomer; Rheocare ATH, INCI: Sodium Polyacrylate (und) Ethylhexyl Stearate (und) Trideceth-6; Rheocare ATC, INCI: Acrylamid / Sodium Acrylate Copolymer (und) Mineralöl (und) Trideceth-6.
Die Polymere können vernetzt oder unvernetzt sein. Vorzugsweise werden vernetzte Polymere eingesetzt.
Erfindungsgemäß bevorzugt sind Polyacrylate und Polyacryloyldimethyltaurate. Besonders bevorzugt sind Polyacrylate, insbesondere deren Natriumsatze, sowie Polyacryloyldimethyltaurate und deren Natrium- und/oder Ammoniumsalze. Die Natriumsalzform ist dabei besonders bevorzugt. Ein erfindungsgemäß besonders bevorzugtes Polymer ist unter dem Namen Cosmedia SP und Cosmedia SPL (Cognis) im Handel. Weiterhin besonders bevorzugt sind die Polyacryloyldimethyltaurat-Zubereitungen Sepigel 305. Simulgel NS. Simulgel EG und Simulgel EPG (siehe vorstehende Erläuterung). Die Polymere werden erfindungsgemäß bevorzugt in einer Gesamtmenge von 0.05 - 1 Gew.-%, besonders bevorzugt 0.1 - 0.8 Gew.-%, insbesondere von 0,2 - 0,6 Gew.-% und ganz besonders bevorzugt von 0.3 - 0.4 Gew.-%, jeweils bezogen auf die Polymeraktivsubstanz in der Gesamtzusammensetzung eingesetzt.

Eine bevorzugte Ausführungsform der erfindungsgemäß verwendeten Öl-in-Wasser-Emulsion enthält (a) 1 - 10 Gew.-% wenigstens eines C16-C18-Esters des Pentaerythrits und/oder des Dipentaerythrits, (b) 1 - 30 Gew.-% wenigstens einer bei 25 °C flüssigen Ölkomponente, (c) 0,05 - 5 Gew.-% wenigstens eines Polyacrylats und (d) 60 - 95 Gew.-% Wasser.
Eine weitere bevorzugte Ausführungsform der erfindungsgemäß verwendeten O/W-Emulsion enthält (a) 1 - 10 Gew.-% wenigstens eines C16-C18-Esters des Pentaerythrits und/oder des Dipentaerythrits, (b) 1 - 30 Gew.-% wenigstens einer bei 25 °C flüssigen Ölkomponente, (c) 0,05 - 5 Gew.-% wenigstens eines Polyacrylats und (d) 40 - 95 Gew.-% Wasser.
Das Polyacrylat (c) ist vorzugsweise ein Natriumpolyacrylat.
Die Ölkomponenten sind auch hier bevorzugt ausgewählt aus den vorstehend beschriebenen Mischungen polarer Öle, darunter besonders bevorzugt Fettsäureester und Dialkylcarbonate, die besonders bevorzugt mit einem Unterschuss unpolarer Öle, wie vorstehend beschrieben, als Gemisch vorliegen.

Eine weitere bevorzugte Ausführungsform der erfindungsgemäß verwendeten Öl-in-Wasser-Emulsion enthält (a) 1 - 10 Gew.-% wenigstens eines C16-C18-Esters des Pentaerythrits und/oder des Dipentaerythrits, (b) 1 - 30 Gew.-% wenigstens einer bei 25 °C flüssigen Ölkomponente, (c) 0,05 - 5 Gew-% wenigstens eines Acryloyldimethyltaurats und (d) 40 - 95 Gew.-% Wasser.
Die Ölkomponenten sind auch hier bevorzugt ausgewählt aus den vorstehend beschriebenen Mischungen polarer Öle, darunter besonders bevorzugt Fettsäureester und Dialkylcarbonate, die besonders bevorzugt mit einem Unterschuss unpolarer Öle, wie vorstehend beschrieben, als Gemisch vorliegen.

Eine weitere bevorzugte Ausführungsform der erfindungsgemäß verwendeten Öl-in-Wasser-Emulsion enthält (a) 1 - 2 Gew.-% wenigstens eines Esters, den man durch Umsetzung des Pentaerythrits und/oder des Dipentaerythrits mit einem Fettsäuregemisch, enthaltend 40 - 50 Gew.-% C16-Fettsäure und 45 - 55 Gew.-% C18-Fettsäure, erhält, (b) 7 - 12 Gew.-% wenigstens einer Ölkomponente, ausgewählt aus bei 25 °C flüssigen Fettsäureestern, Triglyceriden, Dialkylcarbonaten, Kohlenwasserstoffen, Dialkylethern oder einem beliebigen Gemisch dieser Substanzen, (c) 0,5 - 2 Gew-% wenigstens eines Natriumpolyacrylats und (d) 60 - 95 Gew.-% Wasser.
Vorzugsweise sind die unter (a) genannten Ester Pentaerythritester mit folgender Esterverteilung: 12 -19 Gew.-% Monoester, 25 - 35 Gew.-% Diester, 30 - 40 Gew.-% Triester und 6 - 11 Gew-% Tetraester.

Weiterhin wurde überraschend festgestellt, dass die erfindungsgemäß verwendeten Öl-in-Wasser-Emulsionen auch die problemlose Einarbeitung von organischen und/oder anorganischen UV-Filtern ermöglichen, unter gleichzeitiger Lager- und Temperaturstabilität, und dabei ein sehr gutes sensorisches Hautgefühl aufweisen. Antifaltenzusammensetzungen werden von vielen Verbrauchern eingesetzt, um die Folgen der Hautalterung durch schädigende UV-Strahlen zu behandeln. Hier ist es besonders sinnvoll, dem Wunsch des Verbrauchers nach größtmöglichem Lichtschutz nachzukommen und die Antifaltenwirkung mit einem Lichtschutzfaktor zu kombinieren. Erfindungsgemäß besonders bevorzugt verwendete Öl-in-Wasser-Emulsionen sind deshalb dadurch gekennzeichnet, dass sie mindestens eine anorganische und/oder mindestens eine organische UV-Filtersubstanz enthalten.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt. Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3.5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester. 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethyl-hexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI-Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'-methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'-methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.
Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.
Erfindungsgemäß ist mindestens eine organische UV-Filtersubstanz bevorzugt in einer Gesamtmenge von 0,1 - 30 Gew.-%, besonders bevorzugt 0,5 - 20 Gew.-%, außerordentlich bevorzugt 1,0 - 15 Gew.-% und weiter bevorzugt 3,0 - 10 Gew.-%, jeweils bezogen auf die gesamte Zusammensetzung, enthalten.
Erfindungsgemäß ist mindestens eine anorganische UV-Filtersubstanz bevorzugt in einer Gesamtmenge von 0,1 - 15 Gew.-%, besonders bevorzugt 0,5- 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Es kann erfindungsgemäß bevorzugt sein, dass die erfindungsgemäß verwendeten Öl-in-Wasser-Emulsionen mindestens einen nichtionischen Öl-in-Wasser-Emulgator bzw. ein nichtionisches Tensid enthalten, beispielsweise als Solubilisierungsmittel für Parfümöle (siehe oben). Typische Beispiele für nichtionische Öl-in-Wasser-Emulgatoren sind Fettalkoholpolyglycolether, Polyglycerinester, Fettsäurepolyglycolester, Fettsäureamid-polyglycolether, Fettaminpolyglycolether, alkoxylierte Triglyceride, Mischether, Alk(en)yloligoglykoside bzw. Glucoronsäurederivate - gegebenenfalls partiell oxidiert -, Fettsäure-N-alkylglucamide, Polyolfettsäureester, Zuckerester, Sorbitanester, Polysorbate und Aminoxide. Sofern die nichtionischen Öl-in-Wasser-Emulgatoren Polyglycoletherketten enthalten, können diese eine konventionelle, vorzugsweise jedoch eine eingeengte Homologenverteilung aufweisen.

Bevorzugte nichtionische Öl-in-Wasser-Emulgatoren sind ausgewählt aus ethoxylierten C₈-C₂₄-Alkanolen mit durchschnittlich 5 - 100 Mol Ethylenoxid pro Mol, ethoxylierten C₈-C₂₄-Carbonsäuren mit durchschnittlich 5 - 100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen, wobei der gewichtsmittlere HLB-Wert des Öl-in-Wasser-Emulgatorsystems bevorzugt 11 - 17, besonders bevorzugt 12 - 15 und außerordentlich bevorzugt 13 - 14 beträgt. Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 5 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 5 - 100 Mol, vorzugsweise 10 - 30 Mol, Ethylenoxid an technische Fettalkohole mit 12 - 18 Kohlenstoffatomen, wie beispielsweise Kokos-, Palm-, Palmkern- oder Talgfettalkohol, sind geeignet.
Besonders bevorzugte nichtionische Öl-in-Wasser-Emulgatoren sind ausgewählt aus der Gruppe, bestehend aus Ceteth-12, Ceteth-20, Ceteth-30, Steareth-12, Steareth-20, Steareth-30, Laureth-12 und Beheneth-20, sowie Mischungen hiervon.
Die ethoxylierten C₈-C₂₄-Carbonsäuren haben die Formel R¹(OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 5 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 5 - 100 Mol, vorzugsweise 10 - 30 Mol, Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.
Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen. Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C-₁₆-Alkylgruppe an einem Oligoglucosidrest, dessen mittlerer Oligomerisationsgrad bei 1 - 2 liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.
Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.
Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.
Die nichtionischen Öl-in-Wasser-Emulgatoren können u.a. auch durch handelsübliche gelbildende Polymerzubereitungen eingetragen werden, wenn auch nur in geringen Mengen, bezogen auf die erfindungsgemäß verwendete Öl-in-Wasser-Emulsion.

Es kann erfindungsgemäß bevorzugt sein, dass mindestens ein nichtionischer Öl-in-Wasser-Emulgator bzw. ein nichtionisches Tensid in einer Gesamtmenge von 0,2 - 0,7 Gew.-%, bevorzugt 0,3 - 0,7 Gew.-%, besonders bevorzugt 0,4 - 0,7 Gew.-%, außerordentlich bevorzugt 0,5 - 0,6 Gew.-%, jeweils bezogen auf die Gesamtzusammensetzung, enthalten ist.

Zur Verbesserung des Fließverhaltens können ferner Hydrotrope, wie beispielsweise Ethanol, Isopropylalkohol oder Polyole eingesetzt werden. Polyole, die hier in Betracht kommen, besitzen vorzugsweise 2 bis 15 Kohlenstoffatome und mindestens zwei Hydroxylgruppen. Bevorzugt sind diese Polyole ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol. Tripropylenglycol, Diglycerin, Triglycerin, Erythrit, Sorbit Xylitol, sowie Mischungen der vorgenannten Substanzen. Als Polyole geeignete wasserlösliche Polyethylenglycole sind ausgewählt aus PEG-3. PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Erfindungsgemäß bevorzugt verwendete Öl-in-Wasser-Emulsionen sind dadurch gekennzeichnet, dass sie mindestens ein Polyol, ausgewählt aus 1.2-Propylenglycol, 2-Methyl-1,3-propandiol, Glycerin, Butylenglycolen wie 1,2-Butylenglycol. 1,3-Butylenglycol und 1,4-Butylenglycol, Pentylenglycolen wie 1,2-Pentandiol und 1,5-Pentandiol. Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit Xylitol, PEG-3, PEG-4, PEG-6, PEG-7, PEG-8, PEG-9, PEG-10, PEG-12, PEG-14, PEG-16, PEG-18 und PEG-20 sowie Mischungen hiervon, in einer Gesamtmenge von 2 - 20 Gew.-%, bevorzugt 4 - 15 Gew.-%, besonders bevorzugt 5 - 12 Gew.-% und außerordentlich bevorzugt 8 - 10 Gew.-%, jeweils bezogen auf das Gewicht der gesamten Emulsion, enthalten.

Ein weiterer Gegenstand der vorliegenden Anmeldung ist ein nicht-therapeutisches Verfahren zur Reinigung der Haut, insbesondere zur Reinigung der Gesichtshaut, dadurch gekennzeichnet, dass eine gemäß einem der Ansprüche 1 bis 14 verwendete Öl-in-Wasser-Emulsion auf die gegebenenfalls angefeuchtete Haut aufgetragen und nach einer Reinigungsprozedur von 2 bis 200 Sekunden, gegebenenfalls auch länger, abgespült und/oder abgewischt wird, wobei die Applikation auf die Haut mittels eines Tuchs, Pads oder eines ähnlichen Artikels erfolgen kann.

### Weitere fakultative Hilfs- und Zusatzstoffe

Je nach Applikationszweck enthalten die kosmetischen Formulierungen eine Reihe weiterer Hilfs- und Zusatzstoffe wie beispielsweise ionische Emulgatoren/Tenside, weitere Wachs- oder Lipidkomponenten, weitere Verdickungsmittel, Überfettungsmittel, Stabilisatoren, Polymere, Lecithine, Phospholipide, biogene Wirkstoffe, UV-Lichtschufzfaktoren, Antioxidantien, Deodorantien, Filmbildner, Quellmittel, Insektenre-pellentien, Hydrotrope, Solubilisatoren, Konservierungsmittel, Parfümöle, Farbstoffe etc., die nachstehend exemplarisch aufgelistet sind. Die Mengen der jeweiligen Zusätze richten sich nach der beabsichtigten Verwendung.

Als weitere Wachs- oder Lipidkomponenten können Fette und fettähnliche Substanzen mit wachsartiger Konsistenz eingesetzt werden. Die erfindungsgemäß als Fette und fettähnliche Substanzen mit wachsartiger Konsistenz bezeichneten Komponenten weisen inen Schmelzpunkt (unter Normalbedingungen = 1013 mbar) von mindestens 30 °C auf. Hierzu gehören u.a. Triglyceridfette, Mono- und Diglyceride von gesättigten, linearen C12-C40-Fettsäuren, natürliche und synthetische Wachse, Fett- und Wachsalkohole, C10-C40-Fettsäuren, Ester von Fettalkoholen und Fettsäuren, die nicht bei 25°C flüssig sind, sowie Fettsäureamide und beliebige Gemische dieser Substanzen. Unter Fetten versteht man Triacylglycerine, also die Dreifachester von Fettsauren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln.
Erfindungsgemäß einsetzbar und als Konsistenzgeber besonders gut geeignet sind sogenannte gehartete Fette und Öle, die durch Partialhydrierung gewonnen werden. Pflanzliche gehärtete Fette und Öle sind bevorzugt, z. B. gehärtetes Rizinusöl, Erdnussöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.
Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten Triglycerid-Gemische.
Als zusätzliche Wachskomponenten sind insbesondere Mono- und Diglyceride bzw. Mischungen dieser Partialglyceride einsetzbar. Zu den erfindungsgemäß einsetzbaren Glyceridgemischen zählen die von Cognis vermarkteten Produkte Novata AB und Novata B (Gemisch aus C12-C18-Mono-, Di- und Triglyceriden) sowie Cutina MD oder Cutina GMS (Glycerylstearat). Mischester sowie Mischungen aus Mono-, Di- und Triglyceriden sind erfindungsgemäß bevorzugt geeignet, da sie eine geringere Neigung zur Kristallisation zeigen und somit die Performance der erfindungsgemäßen Zusammensetzung weiter verbessern.
Erfindungsgemäß besonders bevorzugt verwendete Öl-in-Wasser-Emulsionen enthalten daher 2 - 10 Gew.-%, bevorzugt 4 - 8 Gew.-%, besonders bevorzugt 5 - 6 Gew.-% mindestens einer Lipidkomponente, ausgewählt aus Glycerylmonostearat, Glyceryldistearat und Mischungen hiervon.

Zu den erfindungsgemäß einsetzbaren Fettalkoholen zählen die linearen C12-C50-Fettalkohole, insbesondere die C12-C24-Fettalkohole, die aus natürlichen Fetten, Ölen und Wachsen gewonnen werden, wie beispielsweise Myristylalkohol. 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte, unverzweigte Fettalkohole. Erfindungsgemäß einsetzbar sind auch Fettalkoholschnitte, wie sie bei der Reduktion natürlich vorkommender Fette und Öle wie z. B. Rindertalg, Erdnussöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß bevorzugt geeignet sind C14-C22-Fettalkohole, die beispielsweise von Cognis unter der Bezeichnung Lanette 16 (C16-Alkohol), Lanette 14 (C14-Alkohol), Lanette O (C16/C18-Alkohol) und Lanette 22 (C18/C22-Alkohol) vermarktet werden.
Fettalkohole verleihen den Zusammensetzungen ein trockeneres Hautgefühl als Triglyceride und sind daher gegenüber letzteren bevorzugt.
Erfindungsgemäß besonders bevorzugte Öl-in-Wasser-Emulsionen enthalten daher 1,5 - 6 Gew.-%, bevorzugt 2 - 5 Gew.-%, besonders bevorzugt 3 - 4 Gew.-% mindestens einer Lipidkomponente, ausgewählt aus Myristylalkohol, Cetylalkohol, Stearylalkohol, Arachidylalkohol, Behenylalkohol und Mischungen hiervon.

Als zusätzliche Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-,Lignocerin-, Cerotin-, Melissin-, Eruca-und Elaeostearinsäure sowie substituierte Fettsäuren, wie z. B. 1 2-Hydroxystearinsäure, und die Amide oder Monoethanolamide der Fettsäuren, wobei diese Aufzählung beispielhaften und keinen beschränkenden Charakter hat.
Erfindungsgemäß verwendbar sind außerdem natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.
Die zusätzliche Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, aus der Gruppe der mit Carbonsäuren veresterten Polyole (andere als Pentaerythrit, Dipentaerythrit oder Tripentaerythrit), insbesondere 1,2-Propylenglycol. Ethylenglycol und Glycerin, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Cetylpalmitat, Stearylstearat, Palmitylstearat, Stearylbehenat, Cetearylbehenat und Behenylbehenat einsetzbar. Aus dieser Gruppe der sogenannten Wachsester ist Cetylpalmitat besonders bevorzugt, insbesondere in Mengen von 0,2 - 1 Gew.-%, bevorzugt 0,4 - 0,8 Gew.-%, besonders bevorzugt 0,5 - 0,6 Gew.-%, jeweils bezogen auf das Gewicht der erfindungsgemäßen Öl-in-Wasser-Emulsion.
Als oberflächenaktive Stoffe können anionische, kationische und/oder amphotere bzw. zwitterionische Tenside bzw. Emulgatoren oder ein beliebiges Gemisch dieser Tenside/Emulgatoren enthalten sein. Der Gehalt oberflächenaktiver Substanzen hängt von der Art der Formulierung ab, übersteigt aber üblicherweise 10 Gew.-% nicht. Eine bevorzugte Ausführungsform der Erfindung enthält keine anionischen oder kationischen Tenside, weist aber einen Gehalt an nichtionischen Tensiden auf.
Die nichtionischen Tenside können u.a. auch in den handelsüblichen Gelbildnern enthalten sein. Typische Beispiele für anionische Tenside sind Seifen, Alkylbenzolsulfonate, Alkansulfonate, Olefinsulfonate, Alkylethersulfonate, Glycerinethersulfonate, alpha-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate und Alkyl(ether)phosphate. Sofern die anionischen Tenside Polyglycoletherketten enthalten, können diese eine konventionelle, vorzugsweise- jedoch eine eingeengte Homologenverteilung aufweisen.
Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze.
Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, alpha-Olefinsulfonate, Ethercarbonsäuren, Alkyloligoglucoside, Fettsäureglucamide, Alkylamidobetaine und/oder Amphoacetate.

Als weitere Verdickungsmittel eignen sich beispielsweise Aerosil-Typen (hydrophile Kieselsäuren), Polysaccharide, insbesondere Xanthan-Gum, Guar-Guar, Agar-Agar, Alginate, Carboxymethylcellulose, Hydroxyethylcellulose und Hydroxypropylcellulose, Polyvinylalkohol, Polyvinylpyrrolidon und Bentonite, wie z. B. Bentone Gel VS-5PC (Rheox).

Weitere optionale Wirkstoffe sind beispielsweise Tocopherol, Tocopherolacetat, Tocopherolpalmitat, Ascorbinsäure, beta-Glucane, Retinol, Bisabolol, Allantoin, Phytantriol, Panthenol, alpha-Hydroxycarbonsäuren (AHA-Säuren), Ceramide, Pseudoceramide, essentielle Öle, Pflanzenextrakte und Vitaminkomplexe.
Als Konservierungsmittel eignen sich beispielsweise Phenoxyethanol, Parabene, Pentandiol oder Sorbinsäure und die in Anlage 6, Teil A und B der Kosmetikverordnung aufgeführten weiteren Stoffklassen.
Als Parfümöle seien genannt Gemische aus natürlichen und synthetischen Riechstoffen. Natürliche Riechstoffe sind Extrakte von Blüten, Stängeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage. Als Farbstoffe können die für kosmetische Zwecke geeigneten und zugelassenen Substanzen verwendet werden. Beispiele sind Cochenillerot A (C.I. 16255). Patentblau V (C.1.42051), Indigotin (C.1.73015), Chlorophyllin (C.1.75810), Chinolingelb (C.1.47005), Titandioxid (C.1. 77891), Indanthrenblau RS (C.1. 69800) und Krapplack (C.1.58000). Diese Farbstoffe werden üblicherweise in Konzentrationen von 0,001 bis 0,1 Gew.-%, bezogen auf die gesamte Mischung, eingesetzt.

Das folgende Beispiel soll den Gegenstand der vorliegenden Anmeldung erläutern, ohne ihn hierauf zu beschränken.

Reinigungscreme / Waschcreme in Form einer O/W-Emulsion mit Anti-Akne-Wirkung und AntiFalten-Wirkung

| | |
|---|---|
| PENTAERYTHRITYL DISTEARAT | 1,200000 |
| COCO-CAPRYLATE/CAPRATE | 4,000000 |
| DICAPRYLYL CARBONATE | 3,000000 |
| PROPYLENE GLYCOL | 5,000000 |
| 1,3-BUTYLENE GLYCOL | 5,000000 |
| SODIUM POLYACRYLATE | 0,500000 |
| PARFUM | 0,350000 |
| RETINYL PALMITATE | 0,050000 |
| 1,10-DECANEDIOL | 0,020000 |
| 10-HYDROXYDECANOIC ACID | 0,020000 |
| SEBACIC ACID | 0,020000 |
| TETRASODIUM EDTA | 0,070000 |
| BHT | 0,050000 |
| PHENOXYETHANOL | 1,000000 |
| METHYLPARABEN | 0,400000 |
| PROPYLPARABEN | 0,400000 |
| Wasser | ad 100,000000 |

Die angegebenen Mengen beziehen sich auf Gew.-% des angegebenen Inhaltsstoffes in der Gesamtzusammensetzung.

2 g der oben dargestellten erfindungsgemäßen Reinigungscreme wurden mit den Fingern auf die angefeuchtete Gesichtshaut, die deutliche Akneprobleme und Hautunreinheiten aufwies, aufgetragen, 60 Sekunden lang einmassiert und dann zunächst mit einem Tuch abgewischt. Die restliche Zusammensetzung wurde mit warmem Wasser abgespült. Die gut gereinigte Haut fühlte sich frisch, zart und spannungs- und irritationsfrei an.

Ebenfalls gute Reinigungsergebnisse wurden erzielt, wenn die Zusammensetzung nicht abgewischt, sondern nur mit Wasser abgespült wurde.

## Patentansprüche

1. Verwendung einer Haut reinigenden Öl-in-Wasser-Emulsion, enthaltend
(a) 1 - 10 Gew.-% wenigstens einer Wachskomponente mit einem Schmelzpunkt von wenigstens 30°C, ausgewählt aus der Gruppe der Pentaerythritester, der Dipentaerythritester und/oder der Tripentaerythritester sowie Mischungen hiervon, wobei die Wachskomponente ausgewählt ist aus der Gruppe der Ester, die durch Umsetzung des Pentaerythrits oder des Dipentaerythrits mit einem Fettsäuregemisch, enthaltend 40 - 50 Gew.-% C16-Fettsäure und 45 - 55 Gew.-% C18-Fettsaure, erhältlich ist.
(b) 1 - 30 Gew.-% wenigstens einer bei 25°C flüssigen Ölkomponente und
(c) 40 - 95 Gew.-% Wasser,
(d) nichtionische Tenside/Emulgatoren in einer Gesamtmenge von 0 - 0,7 Gel.-%, bezogen auf das Gesamtgewicht der Emulsion,
zur Reinigung der Haut, insbesondere zur Reinigung der Gesichtshaut.

2. Verwendung gemäß Anspruch 1, **dadurch gekennzeichnet, dass** die Wachskomponente (a) in einer Menge von 0,2 - 5 Gew.% der Gesamtzusammensetzung enthalten ist.

3. Verwendung gemäß einem der Ansprüche 1 oder 2. **dadurch gekennzeichnet, dass** die Wachskomponente (a) ausgewählt ist aus der Gruppe der Ester des Pentaerythrits mit einem Anteil an (i) 5 - 35 Gew.-% Monoester, (ii) 20 - 50 Gew.-% Diester und (iii) 25 - 50 Gew.-% Triester, und gegebenenfalls Tetraester.

4. Verwendung gemäß einem der Ansprüche 1 bis 3, **dadurch gekennzeichnet, dass** die Wachskomponente (a) ausgewählt ist aus der Gruppe der Ester des Pentaerythrits mit einem Anteil an (i) 12 - 19 Gew.-% Monoester, (ii) 25 - 35 Gew.-% Diester, (iii) 30 - 40 Gew.-% Triester und (iv) 6 - 11 Gew.-% Tetraester.

5. Verwendung gemäß einem der Ansprüche 1 bis 4, **dadurch gekennzeichnet, dass** sie 0,05 - 5 Gew.-% wenigstens eines polymeren Geibildners, ausgewählt aus der Gruppe der Homopolymere oder Copolymere von Acrylsäure und/oder Acrylamid und/oder Acryloyldimelhyltaurat und deren Salzen, enthält. r

6. Verwendung gemäß einem der Ansprüche 1 bis 5, **dadurch gekennzeichnet, dass** die Öl-in-Wasser-Emulsion
(a) 1 - 10 Gew.-% wenigstens eines C16-C18-Esters des Pentaerythrits und/oder des Dipentaerythrits,
(b) 1 - 30 Gew.-% wenigstens einer bei 26 °C flüssigen Ölkomponente
(c) 0,05 - 5 Gew-% wenigstens eines Polyacrylats und
(d) 40 - 95 Gew.-% Wasser
enthält.

7. Verwendung gemäß einem der Ansprüche 1 bis 6, **dadurch gekennzeichnet, dass** die Öl-in-Wasser-Emulsion
(a) 1 - 2 Gew.-% wenigstens eines Esters, den man durch Umsetzung des Pentaerythrits und/oder des Dipentaerythrits mit einem Fettsäuregemisch, enthaltend 40 - 50 Gew.-% C16-Fettsäure und 45 - 55 Gew.-% C18-Fettsäure, erhält,
(b) 7-12 Gew.% wenigstens einer Ölkomponente, ausgewählt aus bei 25 °C flüssigen Fettsäureestern, Triglyceriden, Dialkylcarbonaten, Kohlenwasserstoffen, Dialkylethern oder einem beliebigen Gemisch dieser Substanzen,
(c) 0,5 - 2 Gew-% wenigstens eines Natriumpolyacrylats und
(d) 60 - 95 Gew.-% Wasser
enthält.

8. Verwendung gemäß einem der Ansprüche 1 bis 7, **dadurch gekennzeichnet, dass** die Öl-in-Wasser-Emulsion
(a) 1 - 10 Gew.-% wenigstens eines C16-C18-Esters des Pentaerythrits und/oder des Dipentaerythrits,
(b) 1 - 30 Gew.% wenigstens einer bei 25 °C flüssigen Ölkomponente,
(c) 0,05 - 5 Gew.-% wenigstens eines Acryloyldimethyltaurats und
(d) 40 - 95 Gew.-% Wasser
enthält.

9. Verwendung gemäß einem der Ansprüche 1 bis 8, **dadurch gekennzeichnet, dass** die Ölkomponente mindestens ein polares Öl und mindestens ein unpolares Öl enthält, wobei das Gewichtsverhältnis der Gesamtheit der polaren Öle zur Gesamtheit der unpolaren Öle 5:1 bis 20:1, bevorzugt 6:1 - 10:1 und besonders bevorzugt 7:1 - 8:1 beträgt.

10. Verwendung gemäß einem der Ansprüche 1 bis 9, **dadurch gekennzeichnet, dass** die polaren Öle ausgewählt sind aus Di-n-Octylcarbonat, Coco-caprylate/caprate, Hexyllaurat, Isostearylisononanoat, Isostearylpalmitat, Isostearylstearat, Isostearylisostearat, Caprylic Triglyceride, Capric Triglyceride, Distelöl, Benzoesäure-C12-C15-alkylester, Hexyldecanol, Octyldodecanol, sowie Mischungen dieser Öle, und die unpolare Ölkomponente ausgewählt ist aus Polydimethylsiloxanen (INCI: Dimethicone).

11. Verwendung gemäß einem der Ansprüche 1 bis 10, **dadurch gekennzeichnet, dass** die Öl-in-Wasser-Emulslon 2 - 10 Gew -%, bevorzugt 4 - 8 Gew.-%, besonders bevorzugt 5 - 6 Gew -% mindestens einer Lipidkomponente, ausgewählt aus Glycerylmonostearat, Glyceryldistearat und Mischungen hiervon, enthält.

12. Verwendung gemäß einem der Ansprüche 1 bis 11, **dadurch gekennzeichnet, dass** 1,5 - 6 Gew.-%, bevorzugt 2 - 5 Gew.-%, besonders bevorzugt 3 - 4 Gew.-% mindestens einer Lipidkomponente, ausgewählt aus Myristylalkohol, Cetylalkohol, Stearylelkohol, Arachidylalkohol, Behenylalkohol und Mischungen hiervon, enthalten sind.

13. Verwendung gemäß einem der Ansprüche 1 bis 12, **dadurch gekennzeichnet, dass** keine anionischen und keine kationischen Tenside/Emulgatoren enthalten sind.

14. Verwendung gemäß einem der Ansprüche 1 bis 13, **dadurch gekennzeichnet, dass** nichtionische Tenside/Emulgatoren in einer Gesamtmenge von 0 - 0,5 Gew.-%, besonders bevorzugt 0 - 0,3 Gew.-%, außerordentlich bevorzugt 0 - 0,1 Gew.%, jeweils bezogen auf das Gesamtgewicht der Emulsion, enthalten sind.

15. Nicht-therapeutisches Verfahren zur Reinigung der Haut, insbesondere zur Reinigung der Gesichtshaut, **dadurch gekennzeichnet, dass** eine gemäß einem der Ansprüche 1 bis 14 verwendete Öl-in-Wasser-Emulsion auf die gegebenenfalls angefeuchtete Haut aufgetragen und nach einer Reinigungsprozedur von 2 bis mindestens 200 Sekunden, gegebenenfalls auch länger, abgespült und/oder abgewischt wird, wobei die Applikation auf die Haut mittels eines Tuchs, Pads oder eines ähnlichen Artikels erfolgen kann.

## Claims

1. Use of a skin-cleansing oil-in-water emulsion, containing
(a) 1 to 10% by weight of at least one wax component with a melting point of at least 30°C, selected from the group of pentaerythritol esters, dipentaerythritol esters, and/or tripentaerythritol esters, as well as mixtures thereof, whereby the wax component is selected from the group of esters that can be obtained by reacting the pentaerythritol or dipentaerythritol with a fatty acid mixture, containing 40 to 50% by weight of C16 fatty acid and 45 to 55% by weight of C18 fatty acid,
(b) 1 to 30% by weight of at least one oil component that is liquid at 25°C, and
(c) 40 to 95% by weight of water,
(d) nonionic surfactants/emulsifiers in a total amount of 0 to 0.7% by weight, based on the total weight of the emulsion,
for the cleansing of skin, particularly for the cleansing of facial skin.

2. The use according to claim 1, **characterized in that** the wax component (a) is present in an amount of 0.2 to 5% by weight of the total composition.

3. The use according to one of claims 1 or 2, **characterized in that** the wax component (a) is selected from the group of esters of pentaerythritol with a proportion of (i) 5 to 35% by weight of monoesters, (ii) 20 to 50% by weight of diesters, and (iii) 25 to 50% by weight of triesters, and optionally tetraesters.

4. The use according to one of claims 1 to 3, **characterized in that** the wax component (a) is selected from the group of esters of pentaerythritol with a proportion of (i) 12 to 19% by weight of monoesters, (ii) 25 to 35% by weight of diesters, (iii) 30 to 40% by weight of triesters, and (iv) 6 to 11 % by weight of tetraesters.

5. The use according to one of claims 1 to 4, **characterized in that** it contains 0.05 to 5% by weight of at least one polymeric gelling agent, selected from the group of the homopolymers or copolymers of acrylic acid and/or acrylamide and/or acryloyldimethyl taurate, and the salts thereof.

6. The use according to one of claims 1 to 5, **characterized in that** the oil-in-water emulsion contains
(a) 1 to 10% by weight of at least one C16-C18 ester of pentaerythritol and/or dipentaerythritol,
(b) 1 to 30% by weight of at least one oil component that is liquid at 25°C,
(c) 0.05 to 5% by weight of at least one polyacrylate, and
(d) 40 to 95% by weight of water.

7. The use according to one of claims 1 to 6, **characterized in that** the oil-in-water emulsion contains
(a) 1 to 2% by weight of at least one ester obtained by reacting pentaerythritol and/or dipentaerythritol with a fatty acid mixture, containing 40 to 50% by weight of C16 fatty acid and 45 to 55% by weight of C18 fatty acid,
(b) 7 to 12% by weight of at least one oil component selected from fatty acid esters that are liquid at 25°C, triglycerides, dialkyl carbonates, hydrocarbons, dialkyl ethers, or any mixture of said substances,
(c) 0.5 to 2% by weight of at least one sodium polyacrylate, and
(d) 60 to 95% by weight of water.

8. The use according to one of claims 1 to 7, **characterized in that** the oil-in-water emulsion contains
(a) 1 to 10% by weight of at least one C16-C18 ester of pentaerythritol and/or dipentaerythritol,
(b) 1 to 30% by weight of at least one oil component that is liquid at 25°C,
(c) 0.05 to 5% by weight of at least one acryloyldimethyl taurate, and
(d) 40 to 95% by weight of water.

9. The use according to one of claims 1 to 8, **characterized in that** the oil component contains at least one polar oil and at least one nonpolar oil, whereby the weight ratio of the totality of the polar oils to the totality of the nonpolar oils is 5:1 to 20:1, preferably 6:1 to 10:1, and particularly preferably 7:1 to 8:1.

10. The use according to one of claims 1 to 9, **characterized in that** the polar oils are selected from di-n-octyl carbonate, coco-caprylate/caprate, hexyl laurate, isostearyl isononanoate, isostearyl palmitate, isostearyl stearate, isostearyl isostearate, caprylic triglycerides, capric triglycerides, thistle oil, benzoic acid-C12-C15-alkyl esters, hexyldecanol, octyldodecanol, as well as mixtures of said oils, and the nonpolar oil component is selected from polydimethylsiloxanes (INCI: Dimethicone).

11. The use according to one of claims 1 to 10, **characterized in that** the oil-in-water emulsion contains 2 to 10% by weight, preferably 4 to 8% by weight, particularly preferably 5 to 6% by weight of at least one lipid component selected from glyceryl monostearate, glyceryl distearate, and mixtures thereof.

12. The use according to one of claims 1 to 11, **characterized in that** 1.5 to 6% by weight, preferably 2 to 5% by weight, particularly preferably 3 to 4% by weight of at least one lipid component, selected from myristyl alcohol, cetyl alcohol, stearyl alcohol, arachidyl alcohol, behenyl alcohol, and mixtures thereof are present.

13. The use according to one of claims 1 to 12, **characterized in that** no anionic and no cationic surfactants/emulsifiers are present.

14. The use according to one of claims 1 to 13, **characterized in that** nonionic surfactants/emulsifiers are present in a total amount of 0 to 0.5% by weight, particularly preferably 0 to 0.3% by weight, exceedingly preferably 0 to 0.1% by weight, based in each case on the total weight of the emulsion.

15. A nontherapeutic method for the cleansing of skin, particularly for the cleansing of facial skin, **characterized in that** an oil-in-water emulsion used according to one of claims 1 to 14 is applied to the optionally moistened skin and after a cleansing procedure of 2 to at least 200 seconds, optionally longer as well, is rinsed off and/or wiped off, whereby the application to the skin can occur by means of a cloth, pad, or a similar item.

## Revendications

1. Utilisation d'une émulsion huile-dans-eau de nettoyage de la peau, ladite émulsion contenant
(a) 1 à 10% en poids d'au moins un composant à base de cire ayant un point de fusion d'au moins 30°C, choisi dans le groupe contenant les esters du pentaérythritol, les esters du dipentaérythritol et/ou les esters du tripentaeéythritol et leurs mélanges, le composant à base de cire étant choisi dans le groupe des esters qui sont obtenus par réaction du pentaérythritol ou du dipentaérythritol avec un mélange d'acides gras contenant 40 à 50% en poids d'acides gras à 16 carbones et 45 à 55% en poids d'acides gras à 18 carbones,
(b) 1 à 30% en poids d'au moins un composant huileux liquide à 25°C et
(c) 40 à 95% en poids d'eau,
(d) des tensioactifs/émulsifiants non ionique dans une quantité totale de 0 à 0,7% en poids sur la base du poids total de l'émulsion,
pour le nettoyage de la peau, en particulier pour le nettoyage de la peau du visage.

2. Utilisation selon la revendication 1, **caractérisée en ce que** le composant à base de cire (a) est contenu dans une quantité de 0,2 à 5% en poids de la composition totale.

3. Utilisation selon l'une des revendications 1 ou 2, **caractérisée en ce que** le composant à base de cire (a) est choisi dans le groupe des esters du pentaérythritol comportant un pourcentage (i) de 5 à 35% en poids de monoesters, (ii) de 20 à 50% en poids de diesters et (iii) de 25 à 50% en poids de triester et éventuellement des tétraesters.

4. Utilisation selon l'une des revendications 1 à 3, **caractérisée en ce que** le composant à base de cire (a) est choisi dans le groupe des esters du pentaérythritol comportant un pourcentage (i) de 12 à 19% en poids de monoesters, (ii) de 25 à 35% en poids de diesters, (iii) de 30 à 40% en poids de triesters et (iv) de 6 à 11 % en poids de tétraesters.

5. Utilisation selon l'une quelconque des revendications 1 à 4, **caractérisée en ce qu'**elle comprend de 0,05 à 5% en poids d'au moins un gélifiant polymère choisi dans le groupe des homopolymères ou des copolymères d'acide acrylique et/ou d'acrylamide et/ou d'acryloyldimélhyltaurate et de leurs sels.

6. Utilisation selon l'une des revendications 1 à 5, **caractérisée en ce que** l'émulsion huile-dans-eau contient
(a) 1 à 10% en poids d'au moins un ester en C16 à C18 du pentaérythritol et/ou du dipentaérythritol,
(b) 1 à 30% en poids d'au moins un composant huileux liquide à 25°C,
(c) de 0,05 à 5% en poids d'au moins un polyacrylate et
(d) de 40 à 95% en poids d'eau.

7. Utilisation selon l'une des revendications 1 à 6, **caractérisée en ce que** l'émulsion huile-dans-eau contient
(a) 1 à 2% en poids d'au moins un ester que l'on obtient par réaction du pentaérythritol et/ou du dipentaérythritol avec un mélange d'acides gras contenant 40 à 50% en poids d'acides gras à 16 carbone et de 45 à 56% en poids d'acides gras à 18 carbone,
(b) de 7 à 12% en poids d'au moins un composant huileux choisi parmi les esters d'acides gras liquides à 25°C, les triglycérides, les carbonates de dialkyle, les hydrocarbures, les éthers de dialkyle ou un mélange de ces substnces,
(c) de 0,5 à 2% en poids d'au moins un polyacrylate de sodium et
(d) 60 à 95% en poids d'eau.

8. Utilisation selon l'une des revendications 1 à 7, **caractérisée en ce que** l'émulsion huile-dans-eau contient
(a) de 1 à 10% en poids d'au moins un ester en C16 à C18 du pentaérythritol et/ou du dipentaérythritol,
(b) de 1 à 30% en poids d'au moins un composant huileux liquide à 25°C,
(c) de 0,05 à 5% en poids d'au moins un acryloyldiméthyltaurate et
(d) de 40 à 95% en poids d'eau.

9. Utilisation selon l'une des revendications 1 à 8, **caractérisée en ce que** le composant huileux contient au moins une huile polaire et au moins une huile non polaire, le rapport en poids de la totalité des huiles polaires sur la totalité des huiles non polaires étant de 5:1 à 20:1, de préférence de 6:1 à 10:1 et de façon particulièrement préférée de 7:1 à 8:1.

10. Utilisation selon l'une des revendications 1 à 9, **caractérisée en ce que** les huiles polaires sont choisies parmi le di-n-octylcarbonatee, le coco-caprylate/caprate, le laurate d'hexyle, l'isononanoate d'isostéaryl, le palmitate d'isostéaryle, le stéarate d'isostéaryle, l'isostéarate d'isostéaryle, les triglycérides caprylique, les triglycérides capriques , l'huile de carthame, l'alkylester C₁₂ à C₁₅ de l'acide benzoïque, l'hexyldécanol, l'octyldodécanol, et des mélanges de ces huiles, et le composant huileux non polaire est choisi parmi les polydiméthylsiloxanes (INCI : Diméthicone).

11. Utilisation selon l'une des revendications 1 à 10, **caractérisée en ce que** l'émulsion huile-dans-eau contient de 2 à 10% en poids, de préférence de 4 à 8% en poids, de façon particulièrement préférée de 5 à 6% en poids d'au moins un composant lipidique choisi parmi le monostéarate de glycéryle, le distéarate de glycéryle et des mélanges de ceux-ci.

12. Utilisation selon l'une des revendications 1 à 11, **caractérisée en ce qu'**elle contient 1,5 à 6% en poids, de préférence de 2 à 5% en poids, de manière particulièrement préférée de 3 à 4% en poids d'au moins un composant lipidique choisi parmi l'alcool myristylique, l'alcool cétylique, l'alcool stéarylique, l'alcool arachidique, l'alcool béhénique, et leurs mélanges.

13. Utilisation selon l'une des revendications 1 à 12, **caractérisée en ce qu'**il n'y a pas de tensioactifs/d'émulsifiants anioniques et cationiques.

14. Utilisation selon l'une des revendications 1 à 13, **caractérisée en ce que** les tensioactifs/émulsifiants non ioniques sont contenus dans une quantité totale de 0 à 0,5% en poids, de manière particulièrement préférée de 0 à 0,3% en poids, très préférentiellement de 0 à 0,1% en poids, à chaque fois sur la base du poids total de l'émulsion.

15. Procédé non-thérapeutique de nettoyage de la peau, en particulier pour le nettoyage de la peau du visage, **caractérisé en ce qu'**une émulsion huile-dans-eau, utilisée selon l'une des revendications 1 à 14, est appliquée sur la peau éventuellement humidifiée et est rincée et/ou essuyée après une procédure de nettoyage de 2 à au moins 200 secondes, éventuellement plus, l'application sur la peau pouvant être effectuée à l'aide d'un chiffon, d'un tampon ou d'un article similaire.
